(19) Europäisches Patentamt
European Patent Office
Office européen des brevets

(11) EP 3 187 196 A1

(12) EUROPEAN PATENT APPLICATION

(43) Date of publication:
**05.07.2017 Bulletin 2017/27**

(51) Int Cl.:
***A61L 27/16*** *(2006.01)* ***A61L 27/56*** *(2006.01)*

(21) Application number: **15382675.5**

(22) Date of filing: **29.12.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fundación Tecnalia Research & Innovation**
**20009 San Sebastian (Guipuzcoa) (ES)**

(72) Inventor: **OLALDE GRAELS, Beatriz**
**20009 San Sebastián - Guipúzcoa (ES)**

(74) Representative: **Balder IP Law, S.L.**
**Paseo de la Castellana 93**
**5a planta**
**28046 Madrid (ES)**

(54) **UHMWPE-TYPE POLYMER ARTICLE, PROCESS FOR ITS PRODUCTION AND IMPLANTAND/OR SCAFFOLD MADE THEREOF**

(57) The present invention relates to new ultrahigh molecular weight polyethylene (UHMWPE) articles comprising at least a porous UHMWPE-type polymer structure with an at least trimodal pore distribution and to a process for its preparation which comprises using at least a porogen agent as well as a solvent for generating the porosity. The resulting UHMWPE-type polymer article is well suited for medical implants and/or scaffolds among other applications.

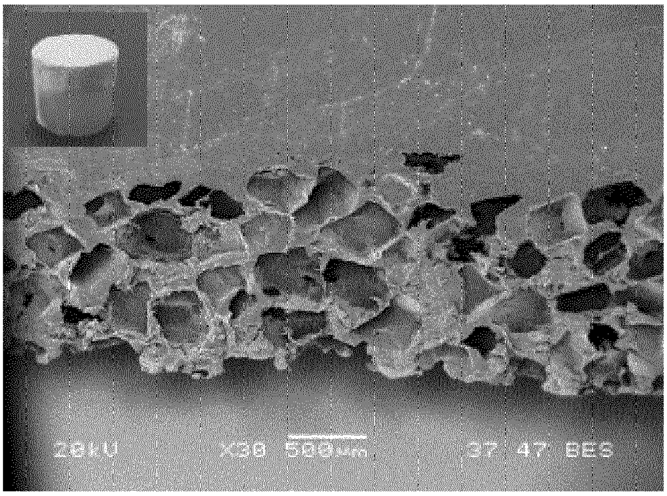


FIG. 14

EP 3 187 196 A1

**Description**

FIELD OF THE INVENTION

The present invention relates to new ultrahigh molecular weight polyethylene (UHMWPE) articles comprising at least a porous UHMWPE-type polymer structure with an at least trimodal pore distribution and to a process for its preparation which comprises using at least a porogen agent as well as a solvent for generating the porosity. The resulting UHMWPE-type polymer article is well suited for medical implants and/or scaffolds among other applications.

BACKGROUND OF THE INVENTION

Ultrahigh molecular weight polyethylene (UHMWPE) is a type of engineering thermoplastic widely used in medical implants because of its chemical inertness, mechanical strength, and low friction coefficient. UHMWPE is most frequently used in endoprostheses of joints.

Presently, UHMWPE is also used to manufacture porous parts of implants. Such porous parts provide fixation to implants because the bone tissue is able to grow into the implants' pores.

The efficacy of the use of porous implants and their reparative capacity are determined by their architectural features. Parameters like pore size and porosity are widely recognized as very important for an optimal osseointegration of the implant. Other architectural features, such as pore shape, pore wall morphology, and interconnectivity between pores, and the presence of bioactive particles are also suggested to be fundamental for cell seeding, migration, growth, mass transport, gene expression, and new tissue formation in three-dimensions. Until now, UHMWPE porous structures have been prepared according to a variety of methods known in the art, which in general present some disadvantages, mainly an inadequate morphology to comply with the above mentioned requirements for their optimal medical application. Consequently, in some instances, the implant may need to be replaced, which can involve additional surgeries with potential complications.

The main difficulties of UHMWPE when it comes to pore structure formation are mainly its high molecular weight and difficult foaming procedure.

Moreover, it has been proven to be advantageous to manufacture implants with a dense part, responsible for the mechanical support, and a porous part responsible for osseointegration. However, as the material used to manufacture each part is usually different, a main problem with this type of implants is the stripping of the superficial porous coating due to insufficient adherence and/or lack of continuity between the two surfaces, porous and dense.

The Thermally Induced Phase Separation Method (TIPS) is commonly used to prepare UHMWPE membranes (see e.g. Ding H., et al., (2007) Journal of Applied Polymer Science, Vol. 105, 3355-3362). The obtained structures show a relatively high porosity, though the pore size is generally not greater than 10 μm and the thickness of the membranes is usually very low.

Another known method for preparing porous UHMWPE structures is compression molding with a porogen. Though a relatively simple method, the high viscosity of the polymer raises difficulties during molding. Moreover, the percentage of porosity is limited by the percentage of porogen used and the pore size by the particle size of the porogen, which negatively influences the interconnectivity of the structure and thus the architectural features needed for bone regeneration. This method allows the inclusion of glass particles, but due to the absence of micro- and nano-porosity in the resulting structures, most of those particles are not exposed. Furthermore, with this method it is very difficult to produce dense-porous parts.

US2010283184 (A1) discloses a medical implant or medical implant part comprising porous ultrahigh molecular weight polyethylene, wherein (i) the ultrahigh molecular weight polyethylene has an average molecular weight of about 400,000 atomic mass units or more, (ii) the ultrahigh molecular weight polyethylene has a porosity of about 15% to about 65%, and (iii) at least about 5% (by volume) of the pores in the ultrahigh molecular weight polyethylene have a diameter of about 200 μm or more. The invention further provides a process for producing a medical implant or medical implant part comprising dispersing a porogen comprising a melt-processable polymer for producing a mixture comprising at least one porogen and ultrahigh molecular weight polyethylene, compressing and melting the mixture contained within a compression mold, and immersing the medical implant or medical implant part obtained in a solvent for a time and under conditions sufficient to extract at least a portion of the porogen from the medical implant or medical implant part. The resulting medical implant or medical implant part shows a structure in which pores are isolated, with limited interconnection and without any type of micro-nano porosity in their walls, as it can be seen from Fig. 3.

Moreover, US2014052264 (A1) discloses a porous implant comprising a plurality of polymeric particles sintered together at a plurality of contact points to form a porous network having pores, the plurality of polymeric particles comprising polyethylene and an antioxidant disposed on a surface of at least some of the polymeric particles and/or in the pores of the porous network. The polyethylene may comprise ultrahigh molecular weight polyethylene. The porous network comprises pores having a diameter of about 15 microns to about 500 microns and a porosity of about 40% to about 90%. Moreover, it discloses that at least some of the polymeric particles have a surface that is about nano-rough to about micro-rough. Additionally, a method of forming the implants is disclosed, the method comprising heating a plurality of polymeric particles in a mold so as to sinter together said

particles at a plurality of contact points, thereby forming a porous network. The nano- to about micro-roughness is, however, not a nano- and micro-porosity as can be seen from fig. 2 Accordingly, a porous network with a structure for optimal osseointegration is not obtained.

WO2014120977 (A1) discloses a UHMWPE membrane that when expanded has a node and fibril structure. The membrane has a porosity of at least 25%, and in exemplary embodiments, the porosity is at least 60%. Additionally, the membrane has a thickness of less than 1 mm, and in some embodiments from about 0,08 to about 0,5 mm, which means that there are no macropores present in the membrane structure. The UHMWPE membrane may be formed by lubricating the UHMWPE polymer, subjecting the lubricated polymer to pressure at a temperature below the melting point of the UHMWPE polymer to form a tape, and expanding the tape at a temperature below the melting temperature of the UHMWPE polymer. Accordingly, macropores with an optimal size for the growth of newly formed bone inside are not obtained.

US2004019389 (A1) discloses a porous surgical implant material for replacing or augmenting tissue in the body comprising a composite material made of an interconnected omni-directional porous matrix structure comprised of synthetic resin and bioactive glass particles distributed throughout said matrix. The synthetic resin may comprise polyethylene. The addition of the bioactive glass particles to the porous polyethylene structure provides for faster fibrovascular ingrowth into implant material. The porous matrix has a porosity of approximately 100 to 500 microns and a pore volume of approximately 40 to 60 percent. The porous surgical implant material is produced by mixing bioglass particles with resin fines to form an admixture, introducing the admixture into a mold, applying heat to mold, whereby the resin fines are sintered together to form a porous implant having bioglass interspersed throughout. With this process, the resulting porous surgical implant only has pores with a size greater than 100 microns, said pores showing no porosity in their walls, a feature which is very important for promoting the cellular adhesion and proliferation.

In spite of the variety of methods, none of the UHMWPE materials obtained accordingly present the adequate morphological and porosity characteristics which allow their successful application in bone tissue engineering. Thus in view of the above there is still the need in the art to provide new biocompatible articles with improved characteristics relative to pore size, pore wall morphology, interconnectivity between pores, and adequate incorporation of bioactive substances among others, which are alleged to be fundamental for cell seeding, migration, growth, mass transport, gene expression, and new tissue formation in three-dimensions, and can thus be successfully used in bone tissue engineering applications. Furthermore, there is also still a need to provide adequate implants comprising a dense-part and a porous part, without the above-mentioned problem, i.e. the stripping of the superficial coating due to insufficient adherence and/or lack of adherence between both parts. SUMMARY OF THE INVENTION

The problem to be solved by the present invention is to provide a new UHMWPE-type polymer article with improved architectural features for optimal osseointegration of the medical implant, and thereby reducing or avoiding unwanted implant replacement surgeries.

The solution is based on that the present inventors have verified that porous UHMWPE-type polymer structures comprising at least a trimodal pore distribution, i.e. with at least macropores, micropores, and nanopores, and pore interconnectivity that resembles natural bone structure, allow for improved osseointegration of the implant and adequate bone regeneration.

Accordingly, a first aspect of the invention relates to an UHMWPE-type polymer article characterized in that it comprises at least a porous UHMWPE-type polymer structure, said structure comprising an at least trimodal pore distribution as follows:

(i) a pore distribution A corresponding to pores A with an average size between 50 μm and 500 μm which are interconnected throughout the whole article;
(ii) a pore distribution B corresponding to the voids between adjacent pores A of an average size between 5 μm and 70 μm referred to as pores B; and
(iii) a pore distribution C corresponding to pores C of an average size of less than about 10μm which are located in the walls of the pores A and pores B.

In a second aspect the invention relates to a process for the production of an UHMWPE-type polymer article according to claims 1 to 5, characterized in that it comprises the following steps:

a) contacting the UHMWPE-type polymer with at least one solvent and at least one porogen, wherein the percentage of UHMWPE-type polymer is between 5% to 50% by weight in respect to the solvent,
b) mixing a UHMWPE-type polymer with at least one solvent and at least one porogen until a homogeneous mixture is obtained,
c) heating the mixture obtained in b) to a temperature of about 30°C to about 50°C above the cloud-point temperature of the UHMWPE-type polymer/solvent/porogen mixture until complete dissolution of the UHMWPE-type polymer in the solvent,
d) cooling the mixture obtained in c) to a temperature at which phase separation occurs,
e) forming said cooled mixture into a shaped intermediate article,
f) removing the solvent and the porogen agent, and
g) recovering the article comprising an UHMWPE-type polymer.

In a third aspect the invention relates to an implant and/or scaffolds characterized in that it comprises

an UHMWPE-type polymer article according to claims 1 to 5.

BRIEF DESCRIPTION OF THE DRAWING

FIG. 1 - SEM micrographs of porous UHMWPE-type polymer structures produced with a porogen with different particle sizes: 120-180 μm (left) and 250-350 μm (right).

FIG. 2 - SEM micrographs of porous UHMWPE-type polymer structures produced with different percentages of UHMWPE-type polymer: 10% by weight (top), 13% by weight (left) and 17% by weight (right).

FIG. 3 - SEM micrographs of porous UHMWPE-type polymer structures produced with different solvents: diphenyl ether (left) and liquid paraffin (right).

FIG. 4 - SEM micrographs of porous UHMWPE-type polymer structures produced with different types of porogens: sucrose (left) and NaCl (right).

FIG. 5 - SEM micrographs of porous UHMWPE-type polymer structures produced with 50% by weight of hydroxyapatite (HA) and different solvents: diphenyl ether (left) and liquid paraffin (right).

FIG. 6 - SEM micrographs of the walls of the porous UHMWPE-type polymer structures with different types of calcium phosphate particles: HA 50-80 μm (left) and β-TCP 2-5 μm (right).

FIG. 7 - EDS spectra of HA particles (left) and HA particles incorporated in the porous UHMWPE-type polymer structure (right).

FIG. 8 - EDS spectra of β-TCP particles (left) and β-TCP particles incorporated in the porous UHMWPE-type polymer structure (right).

FIG. 9 - SEM micrographs of porous UHMWPE-type polymer structures produced with 17% by weight of UHMWPE-type polymer, liquid paraffin and NaCl particles within a size range of 250-350 μm.

FIG. 10 - SEM micrographs of porous UHMWPE-type polymer structures produced with 13% by weight of UHMWPE-type polymer, diphenyl ether and NaCl particles within a size range of 350-420 μm.

FIG. 11 - SEM micrographs and EDS spectra of porous UHMWPE-type polymer structures produced with 17% by weight of UHMWPE-type polymer, diphenyl ether, NaCl particles within a size range of 350-420 μm and HA particles.

FIG. 12 - SEM micrographs of porous UHMWPE-type polymer structures produced with 13% by weight of UHMWPE-type polymer, liquid paraffin, NaCl particles within a size range of 350-420 μm and β-TCP particles.

FIG 13 - EDS spectra of β-TCP particles (left) and β-TCP particles incorporated in the porous UHMWPE-type polymer structure.

FIG 14 - SEM micrograph of a dense-porous UHMWPE-type polymer article.

FIG. 15 - SEM micrographs of porous UHMWPE-type polymer structures produced with 13% by weight of UHMWPE-type polymer, liquid paraffin and KCl particles within a size range of 112-180 μm.

FIG. 16 - SEM micrographs of UHMWPE foams prepared with 10% by weight UHMWPE, liquid paraffin and Sucrose particles in the size range from 250 to 350μm.

FIG. 17 - Image of a cylindrical dense-porous UHMWPE-type polymer sample.

FIG. 18 - SEM micrographs of a cylindrical dense-porous UHMWPE-type polymer article.

DETAILED DESCRIPTION OF THE INVENTION

<u>The UHMWPE- type polymer article</u>

As mentioned above the new UHMWPE-type polymer articles according to the invention present a new and very characteristic pore morphology and pore distributions which makes them very suitable for applications such as tissue engineering.

The pore distribution A is generated during the step f) of the process of the invention when the porogen agent is removed leaving the pores A which retain the shape of the porogen agent, and which are located within and throughout the whole UHMWPE-type polymer structure. Moreover, as shown in Fig. 1 the size range of these pores A corresponds to the size range of the selected porogen. These pores A allow for an optimal newly formed bone tissue invasion.

The pores B of the pore distribution B are originated as a result of the location of the porogen agent particles in the intermediate shaped article obtained in step e). The porogen agent particles are located adjacent and in contact and when the particles are removed during step f), they leave voids between adjacent pores A.

The pore distribution C is generated due to the presence of the solvent in the intermediate shaped article obtained in step e). When the solvent is eliminated in

step f) it originates pores of about 10 μm or smaller, or even smaller than 1 μm, which will be referred hereinafter as pores C. These pores C can be in the nanometric range or both in the micro- and nanometric ranges and are located in the walls of pores A and pores B. This micro- nano-porosity facilitates cellular adhesion and nutrient transport.

These three pore distributions may be clearly observed for example in Fig. 10.

In a preferred embodiment of the invention the pores occupy 70 to 95% of the total volume of the UHMWPE-type polymer article. Preferably, the pores occupy 75 to 95% of the total volume of the article.

In another preferred embodiment the UHMWPE-type polymer article further comprises at least one bioactive particle within the porous UHMWPE-type polymer structure, selected from the group of metallic particles, ceramic particles or mixtures thereof. These bioactive particles should remain intact throughout the whole production process of the UHMWPE-type polymer article, including during the steps of removing the solvent and the porogen agent, so as to remain incorporated in the porous structure of the UHMWPE-type polymer article and accessible for cellular recognition. Examples of suitable bioactive particles according to the invention, but not limited to, are titanium particles, calcium phosphates, preferably selected from tricalcium phosphate, hydroxyapatite, biphasic calcium phosphate and mixtures thereof. Moreover, the bioactive particles may have different geometries and sizes. The incorporation of bioactive particles in the porous UHMWPE-type polymer structure of the UHMWPE-type polymer article according to the invention can be seen in Figs. 5 and 6.

In yet another preferred embodiment of the present invention the UHMWPE-type polymer article may further comprise a dense UHMWPE-type polymer structure to form a dense-porous UHMWPE-type polymer article. The dense UHMWPE-type polymer structure provides for mechanical resistance, while the porous UHMWPE-type polymer structure provides for the bone tissue incorporation. As shown in Figs. 17 and 18, the porous UHMWPE-type polymer structure is created on a dense UHMWPE-type polymer surface. A continuity of the material and an optimal interface can be appreciated, which avoids the stripping of both parts.

A skilled person may appreciate that according to the invention it is possible to produce porous UHMWPE-type polymer articles or dense-porous UHMWPE-type polymer articles with complex geometries, and unlimited and homogeneous thickness of the porous part.

<u>Process for the production of an UHMWPE-type polymer article</u>

The above described characteristic and advantageous at least trimodal pore distribution and morphology of the UHMWPE-type polymer articles of the present invention is achieved according to the process of the invention by means of combining at least one porogen agent and at least one solvent. Thus, the process of the invention provides improved UHMWPE-type polymer articles which can, among other applications, be used in medical implants and/or scaffolds as it is disclosed further below.

According to the invention, porogen particles are sieved to select the desired size ranges. Then the UHMWPE-type polymer and the solvent are weight in a desired proportion. The proportion of UHMWPE-type polymer and solvent will influence the obtained porosity, in particular the porosity present in the walls of pores A and pores B, and also the mechanical properties of the UHMWPE-type polymer article. Subsequently, the UHMWPE-type polymer, the porogen and the solvent are introduced into a glass reactor. An amount of antioxidant sufficient to prevent oxidation of the UHMWPE-type polymer during the process is added. The reactor is sealed and an inert atmosphere for preventing the degradation of any of the components is introduced. Then the components UHMWPE-type polymer/porogen/solvent are mixed under mechanical agitation, and heated to a temperature of about 30°C to about 50°C above the cloud-point temperature of the system for about 30 minutes. At this temperature, a transparent and homogeneous solution is obtained. This temperature directly depends on the solvent used as well as on the percentage of UHMWPE-type polymer used. In a preferred embodiment of the present invention this temperature ranges between 150°C and 200°C. Subsequently, the reactor is cooled with air or in a water bath, so that the separation of phases is induced and the system solidifies. Once the mixture UHMWPE-type polymer/porogen/solvent is cooled, it is cut into a shaped intermediate article. The porogen and the solvent are removed by using solvents that dissolve each of these components. Usually, the porogen is removed with water and the solvent with ethanol or acetone. As mentioned above, the removal of the porogen results in the formation of pores A and B, while the removal of the solvent originates pores C.

*Ultrahigh Molecular Weight Polyethylene-type (UHMWPE-type) polymer*

Ultrahigh Molecular Weight Polyethylene-type (UHMWPE-type) polymers refer to linear polyethylene with a molecular weight in the range of 3,000,000 to 6,000,000 of formula

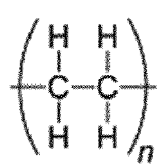

wherein n is greater than 100 000.

Examples of UHMWPE-type polymers within

the scope of the present invention include, but are not limited to, cross-linked UHMWPE, UHMWPE stabilized with an antioxidant, for example, vitamin E-stabilized UHMWPE or vitamin E-highly cross-linked UHMWPE. UHMWPE-type polymers for use in the present invention are commercially available and/or can be obtained by synthesis processes well known in the art.

As mentioned above, the proportion between the percentage of UHMWPE-type polymer and the solvent used determines the porosity of the porous UHMWPE-type polymer structure, in particular the porosity present in the walls of pores A of the pore distribution A and of pores B of the pore distributions B. The lower the percentage of UHMWPE-type polymer used, the higher is the porosity of the porous UHMWPE-type polymer structure. In a preferred embodiment of the present invention the percentage of UHMWPE-type polymer is between 10% to 30% by weight with respect to the solvent.

Fig. 2 clearly shows that the porosity of the porous UHMWPE-type polymer structure is inversely proportional to the percentage of UHMWPE-type polymer used. As it can be seen from the SEM micrographs, the porous UHMWPE-type polymer structure produced using 10% by weight of UHMWPE-type polymer with respect to the solvent shows a higher porosity in its structure than the porous UHMWPE-type polymer structure produced using 17% by weight of UHMWPE-type polymer.

*Solvent*

Suitable solvents according to the invention have to dissolve the UHMWPE-type polymer at a given temperature for obtaining a transparent and homogeneous solution. Subsequently, the cooling of said solution has to result in a separation of phases: a phase rich in polymer that will form the skeleton of the porous structure and a phase poor in polymer that will provide for the micro- and nano-pores of the porous structure. Moreover, the solvent shall not dissolve the porogen. Examples of suitable solvents according to the invention, but not limited to, are diphenyl ether, diisodecyl phthalate, liquid paraffin, polytetramethylene glycol, dioctyl phthalate, soybean oil, decalin, mineral oil or mixtures thereof.

The selected solvent influences the porosity of the porous UHMWPE-type polymer structure. Porous UHMWPE-type polymer structures produced with the same percentage of UHMWPE-type polymer and size of porogen particles, but with different solvents show different porosity as shown in Fig. 3.

*Porogen*

As referred above porogen particles originate pores A of the pore distribution A, which have a direct influence on the cellular behaviour.

Pores A of the pore distribution A have a similar aspect to the selected porogen particles that originated them and the size range of these pores A corresponds to the size range of the particles of the selected porogen that originated them.

According to a particular embodiment of the present invention, at least two different porogen agents, not necessarily differing in their chemical nature, but differing in their particle size distributions may be used. Thus, a first porogen agent having a first size distribution and a second porogen agent having a second size distribution, are simultaneously used for generating two different pore distributions A, that is a first pore distribution A and a second pore distribution A' in the obtained porous article. Both pore distributions A and A' correspond to pores with an average size comprised between 50-500 μm. The shape of the pores A' can be different from the shape of pores A.

Fig. 1 shows that porous UHMWPE-type polymer structures produced with porogen particles in the size range of 120 μm to 180 μm (left) have smaller pores A than porous UHMWPE-type polymer structures produced with porogen particles in the size range of 250 μm to 350 μm (right).

Fig. 4 shows porous UHMWPE-type polymer structures produced under the same conditions, but with porogens of different nature. The left hand porous UHMWPE-type polymer structure was produced using a sugar-type porogen, namely sucrose with particles in the size range between 250 μm to 350 μm, and the right hand porous UHMWPE-type polymer structure was produced using a salt-type porogen, namely sodium chloride with particles in the same size range as above. As it can be observed pores A of the porous UHMWPE-type polymer structure produced with sucrose have a similar monoclinic geometry to that of sucrose particles, while the pores A of the porous UHMWPE-type polymer structure produced with NaCl have a cubic geometry similar to that of NaCl particles.

Suitable porogens according to the invention shall not suffer any change during the production process, i.e., they shall not melt at working temperatures nor dissolve in the solvent used. Examples of suitable porogens according to the invention, but not limited to, are salt-type porogens, sugar-type porogens or mixtures thereof.

Preferred salt-type porogens according to the invention are selected from sodium chloride, sodium citrate, sodium, sodium tartrate, potassium chloride or mixtures thereof.

Preferred sugar-type porogens according to the invention are selected from sucrose, galactose or mixtures thereof.

*Bioactive particles*

In a preferred embodiment of the invention the UHMWPE-type polymer of step a) may be further contacted with at least one type of bioactive particles. The bioactive particles can be selected from the group of metallic particles, ceramic particles or mixtures thereof.

In this respect, it is of upmost relevance the porosity of the porous UHMWPE-type polymer structures according to the present invention. Firstly, because it facilitates cellular adhesion due to its topography, and secondly because it provides for cellular recognition due to the accessibility of the incorporated bioactive particles. According to the present invention, the incorporated bioactive particles remain trapped in the UHMWPE-type polymer structure, but visible being directly accessible. Moreover, the size range of the incorporated bioactive particles can be very broad, including particles in the nanometric size ranges, similar to the ceramic component of natural bone, without escaping from the porous structure.

Fig. 5 shows a uniform distribution of hydroxyapatite (HA) particles in the size range between 50 $\mu$m and 80 $\mu$m incorporated in the porous UHMWPE-type polymer structure according to the invention and produced under the same conditions, but with different solvents. As it can be observed the bioactive ceramic particles remain incorporated and accessible in the porous UHMWPE-type polymer structure, independently of the solvent used.

On the other hand, it is possible to incorporate, in a very effective manner, bioactive particles of different nature and size in the porous UHMWPE-type polymer structure by keeping the original shape of said bioactive particles.

The SEM micrographs of the walls of the porous UHMWPE-type polymer structures with different type and size of particles (Fig. 6) show that once incorporated in the porous UHMWPE-type polymer structure, the bioactive particles remain trapped and accessible, while keeping their shape.

Moreover, through the EDS analysis of the bioactive particles incorporated in the porous UHMWPE-type polymer structures according to the invention, it is possible to qualitatively confirm that the composition of said bioactive particles remains unchanged (Fig. 7 and 8).

Suitable bioactive particles according to the invention shall remain intact throughout the whole process of production of the UHMWPE-type polymer article, including during the steps of removing the solvent and the porogen agent, so as to remain incorporated in the porous structures of the UHMWPE-type polymer article and accessible for cellular recognition.

Examples of suitable bioactive particles according to the invention include, but are not limited to, titanium particles and calcium phosphates, preferably selected from tricalcium phosphate, hydroxyapatite, biphasic calcium phosphate or mixtures thereof.

*Dense porours UHMWPE-type polymer article*

In a preferred embodiment of the process according to the invention, a dense-porous UHMWPE-type polymer article may be manufactured. This may be achieved by introducing at least one surface of a dense UHMWPE-type polymer structure in the UHMWPE-type polymer/solvent/porogen mixture after heating said mixture until a complete dissolution of the UHMWPE-type polymer (step c) and before cooling said mixture to a temperature at which phase separation occurs (step d). A porous UHMWPE-type polymer structure is; thereby, created on the surface of the dense UHMWPE-type polymer structure, forming an optimal continuous interface as can be observed from Figs. 14 and 18. In the dense-porous UHMWPE-type polymer article, the dense UHMWPE-type polymer structure provides for mechanical resistance, while porous UHMWPE-type polymer structure provides for bone tissue incorporation.

*Implant and/or scaffolds*

The resulting variety of UHMWPE-type polymer articles of the present invention may be used for many different applications, such as implants, prosthesis or tissue engineering scaffolds, due to the UHMWPE-type polymer biocompatibility, cell culture matrices, controlled release matrices, wound dressings, separation membranes, column fillers of chromatography, filters, packaging and insulating materials, among others.

According to their intended use, the articles may present different forms such as membranes, cylinders, prisms, etc. Moreover, porous articles may be further processed if necessary, according to conventional techniques, such as cutting, to further adjust shape or size to the desired application.

Additionally, the UHMWPE-type polymer may subsequently be subjected to a cross-linking treatment and/or to the incorporation of an antioxidant, such as for example vitamin E, for controlling the cross-linking process.

Depending on the intended use of an article, parameters such as porosity, pore size distribution, size and shape of said article, its composition, for instance the presence and the concentration of certain bioactive particles, among others are well-designed and controlled.

It can be appreciated that the implants and/or tissue engineering scaffolds comprising an UHMWPE-type polymer article according to the invention may form part of a kit.

EXAMPLES

The characterization of the UHMWPE-type polymer articles according to the invention was performed as follows:

Porosity was defined as the volume of the pores divided by the total volume of the porous UHMWPE-type polymer structure. The UHMWPE-type polymer structure was dipped in pure alcohol for 10 h. Then,

the UHMWPE-type polymer structure was taken out, and the alcohol on the surface of the UHMWPE-type polymer structure was softly wiped up with filter paper. Finally, the UHMWPE-type polymer structure was weighed quickly. The formula for porosity was as follows:

$$\text{Porosity} = \frac{(W_0 - W)\bar{\rho}}{\bar{\rho}W_0 + (\rho - \bar{\rho})W} \times 100$$

where W is the total weight of the dry porous UHMWPE-type polymer structure, $W_0$ is the weight of the wet porous UHMWPE-type polymer structure; p is the polymer density, and p is the absolute alcohol density. The porosity data were obtained by averaging over five specimens.

Microstructure of the sample was evaluated by scanning electron microscopy (SEM). The porous UHMWPE-type polymer structure was fractured in liquid nitrogen and then sputtered with gold to observe the morphology of the cross section by SEM. (JEOL JSM 5910-LV (20 kV)). The image analysis coupled with Energy Dispersive Spectrometry (EDS) analysis on a SEM was applied to the characterization of the elemental composition of the particles observed in the SEM images.

**EXAMPLE 1- UHMWPE-Type polymer article prepared with 17wt% UHMWPE, liquid paraffin and NaCl particles in the size range from 250 to 350 μm**

First, sodium chloride (NaCl) (Sigma Aldrich) particles were sieved with a diameter ranging from 250-350 μm. 1.8 g of UHMWPE, 8.55 ml of liquid paraffin (LP) (Merk), 0.1 g of Irganox 1010 (Sigma Aldrich) and 8.1 g of NaCl particles were put in a glass reactor and mechanically mixed at 100 rpm (Janke & Kunkel RW-20). After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. Then, the glass reactor was cool down at room temperature to solidify. The solidified NaCl/UHMWPE/LP mixture was removed from the glass reactor and was cut into pieces having a cylindrical dimension of 10mm diameter and 15mm length. Each piece was immersed in 25mL of n-hexane and shaken under orbital agitation at room temperature for 24 h (n-hexane was changed every 12 h) to extract the LP. Then, the piece was immersed in 25 mL of distilled water to extract the NaCl. These two processes were alternately run during 15 days. Finally, the porous UHMWPE sample was freeze-dried to dry completely.

RESULTS EXAMPLE 1

The porosity of the UHMWPE was 79%.

Larger pores between 250-350 μm, retaining the shapes of the original NaCl particles, were observed in the SEM image (Fig. 9). Moreover, in the wall of those pores, micro and nanopores were detected due to the liquid paraffin elimination. And finally, pores around 20 μm were appreciated that match to the size of the opening between the pores.

**EXAMPLE 2 - UHMWPE-Type polymer article prepared with 13wt% UHMWPE, diphenyl ether and NaCl particles in the size range from 350 to 420 μm**

First, sodium chloride (NaCl) (Sigma Aldrich) particles were sieved with a diameter ranging from 350-420 μm. 1.35 g of UHMWPE, 9 ml of diphenyl ether (DE) (Sigma Aldrich), 0.1 g of Irganox 1010 (Sigma Alduch) and 8.1 g of NaCl particles were put in a glass reactor and mechanically mixed at 100 rpm. After the glass reactor was full of nitrogen, it was sealed. Then, the glass reactor was put in an electric heating and the mixture was heated to 200°C (30-50°C above the cloud-point temperature of the solution) for 30 min until the UHMWPE was dissolved in the DE completely. Then, the glass reactor was cool down at room temperature to solidify. The solidified NaCl/UHMWPE/DE mixture was removed from the glass reactor and was cut into pieces having a cylindrical dimension of 10mm diameter and 15mm length. Each piece was immersed in 25mL of acetone and shaken under orbital agitation at room temperature for 24 h (acetone was changed every 12 h) to extract the DE. Then, the piece was immersed in 25 mL of distilled water to extract the NaCl. These two processes were alternately run during 15 days. Finally, the porous UHMWPE sample was freeze-dried to dry completely.

RESULTS EXAMPLE 2

The porosity of the UHMWPE was 92%.

Larger pores between 350-420 μm, retaining the shapes of the original of NaCl particles, were observed in the SEM image (Fig. 10). Moreover, in the wall of those pores, micro and nanopores were detected due to the diphenyl ether elimination. And finally, pores around 20 μm were appreciated that match to the size of the opening between the pores.

**EXAMPLE 3 - UHMWPE-Type polymer article prepared with 17wt% UHMWPE, diphenyl ether, NaCl particles in the size range from 350 to 420 μm and HA particles**

First, sodium chloride (NaCl) (Sigma Aldrich) particles were sieved with a diameter ranging from 350-420 μm. 1.35 g of UHMWPE , 9 ml of diphenyl ether (DE) (Merk), 0.1 g of Irganox 1010 (Sigma Aldrich), 0.675

g of HA (Teknimed) and 8.1 g of NaCl particles were put in a glass reactor and mechanically mixed at 100 rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. Then, the glass reactor was cool down at room temperature to solidify. The solidified NaCl/UHMWPE/HA/DE mixture was removed from the glass reactor and was cut into pieces having a cylindrical dimension of 10mm diameter and 15mm length. Each piece was immersed in 25mL of acetone and shaken under orbital agitation at room temperature for 24 h (acetone was changed every 12 h) to extract the DE. Then, the piece was immersed in 25 mL of distilled water to extract the NaCl. These two processes were alternately run during 15 days. Finally, the porous UHMWPE sample was freeze-dried to dry completely.

RESULTS EXAMPLE 3

The porosity of the UHMWPE was 78%.

Fig. 11 (top) shows larger pores, retaining the shapes of the original NaCl particles of 350-420 μm. Inter pore opening size of about 50 μm was detected in the bonding of the pores. Moreover, in the wall of those pores, micro and nanopores were detected due to the diphenyl ether elimination.

HA particles can be also appreciated, with a size around 50-80 μm, randomly distributed in and inside skeleton of the porous UHMWPE-type polymer structure. These particles were successfully integrated into the matrix with no visible agglomeration formation. From the EDS characterization (Fig. 11 bottom) can be confirmed that the particles observed in the SEM images were, basically, composed by calcium and phosphorous elements, two of the mayor components of HA.

**EXAMPLE 4 - UHMWPE-Type polymer article prepared with 13wt% UHMWPE, liquid paraffin, NaCl particles in the size range from 350 to 420 μm and β-TCP particles**

First, sodium chloride (NaCl) (Sigma Aldrich) particles were sieved with a diameter ranging from 350-420 μm. 1.35 g of UHMWPE , 9 ml of liquid paraffin (LP) (Merk), 0.1 g of Irganox 1010 (Sigma Aldrich), 0.675 g of β-TCP (CamBioceramics) and 8.1 g of NaCl particles were put in a glass reactor and mechanically mixed at 100 rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. Then, the glass reactor was cool down at room temperature to solidify. The solidified NaCl/UHMWPE/β-TCP/LP mixture was removed from the glass reactor and was cut into pieces having a cylindrical dimension of 10mm diameter and 15mm length. Each piece was immersed in 25mL of n-hexane and shaken under orbital agitation at room temperature for 24 h (n-hexane was changed every 12 h) to extract the LP. Then, piece was immersed in 25 mL of distilled water to extract the NaCl. These two processes were alternately run during 15 days. Finally, the porous UHMWPE sample was freeze-dried to dry completely.

RESULTS EXAMPLE 4

The porosity of the UHMWPE was 70%.

Fig. 12 shows larger pores, retaining the shapes of the original NaCl particles of 350-420 μm. Inter pore opening size of about 10 μm was detected in the bonding of the pores. Moreover, in the wall of those pores, micro and nanopores were detected due to the liquid paraffin elimination.

β-TCP particles can be also appreciated, with a size around 2-5 μm, randomly distributed in and inside skeleton of the porous UHMWPE-type polymer structure. These particles were successfully integrated into the matrix with no visible agglomeration formation. From the EDS characterization (Fig. 13) can be confirmed that the particles observed in the UHMWPE has the same spectrum of raw particles.

**EXAMPLE 5 - Dense-porous UHMWPE-type polymer article**

First, sodium chloride (NaCl) (Sigma Aldrich) particles were sieved with a diameter ranging from 350-420 μm. 1.35 g of UHMWPE, 9 ml of liquid paraffin (LP) (Merk), 0.1 g of Irganox 1010 (Sigma Aldrich) and 8.1 g of NaCl particles were put in a glass reactor and mechanically mixed at 100rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. After that, the UHMWPE dense bar with dimensions of 15mm diameter and 50mm length (GoodFellow) was introduced 2mm depth in the NaCl/UHMWPE/LP mixture and was maintained for 10 min. Then, the glass reactor was cool down at room temperature to solidify. The NaCl/UHMWPE/LP mixture was solidified over the part of UHMWPE dense bar that was introduced in NaCl/UHMWPE/LP solution. The solidified NaCl/UHMWPE/LP mixture and UHMWPE dense bar was removed from the glass reactor and was machined at 10mm diameter. Finally, the sample was immersed in 25mL of n-hexane and shaken under orbital agitation at room temperature for 24 h (n-hexane was changed every 12 h) to extract the LP. Then, piece was immersed in 25 mL of distilled water to extract the NaCl. These two processes were alternately run during 15 days. The porous UHMWPE sample was freeze-dried to dry completely and porous-dense UHMWPE article was obtained.

RESULTS EXAMPLE 5

From the SEM image (Fig. 14) it can be observed that porous UHMWPE-type polymer structure was created on the dense UHMWPE surface. It can be appreciated a continuity of the material.

**EXAMPLE 6 - UHMWPE-Type polymer article prepared with 13wt% UHMWPE, liquid paraffin and KCl particles in the size range from 112 to 180 μm**

First, potassium chloride (KCl) (Sigma Aldrich) particles were sieved with a diameter ranging from 180-250 μm. 1.8 g of UHMWPE, 8.55 ml of liquid paraffin (LP) (Merk), 0.1 g of Irganox 1010 (Sigma Aldrich) and 8.1 g of KCl particles were put in a glass reactor and mechanically mixed at 100 rpm (Janke & Kunkel RW-20). After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. Then, the glass reactor was cool down at room temperature to solidify. The solidified KCl/UHMWPE/LP mixture was removed from the glass reactor and was cut into pieces having a cylindrical dimension of 10mm diameter and 15mm length. Each piece was immersed in 25mL of n-hexane and shaken under orbital agitation at room temperature for 24 h (n-hexane was changed every 12 h) to extract the LP. Then, the piece was immersed in 25 mL of distilled water to extract the KCl. These two processes were alternately run during 15 days. Finally, the porous UHMWPE sample was freeze-dried to dry completely.

RESULTS EXAMPLE 6

The porosity of the UHMWPE was 79%.

Larger pores between 112-180 μm, retaining the shapes of the original of KCl particles, were observed in the SEM image (Fig. 15). Moreover, in the wall of those pores, micro and nanopores were detected due to the liquid paraffin elimination. And finally, pores around 50 m were appreciated that match to the size of the opening between the pores.

**EXAMPLE 7 - UHMWPE-Type polymer article prepared with 17wt% UHMWPE, liquid paraffin and sucrose particles in the size range from 250 to 350 μm**

First, sucrose (SA) (Scharlab) particles were sieved with a diameter ranging from 250-350 μm. 1 g of UHMWPE, 9 ml of liquid parafine (LP) (Sigma Aldrich), 0.1 g of Irganox 1010 (Sigma Aldrich), 0 8.1 g of SA particles were put in a glass reactor and mechanically mixed at 100 rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. Then, the glass reactor was cool down at room temperature to solidify. The solidified SA/UHMWPE/LP mixture was removed from the glass reactor and was cut into pieces having a cylindrical dimension of 10mm diameter and 15mm length. Each piece was immersed in 25mL of n-hexane and shaken under orbital agitation at room temperature for 24 h (n-hexane was changed every 12 h) to extract the LP. Then, the piece was immersed in 25 mL of distilled water to extract the SA. These two processes were alternately run during 15 days. Finally, the porous UHMWPE sample was freeze-dried to dry completely.

Figure 16 shows SEM micrographs of UHMWPE foams prepared with 10% by weight UHMWPE, liquid paraffin and Sucrose particles in the size range from 250 to 350μm.

**EXAMPLE 8 - Cylindrical dense-porous UHMWPE-type polymer article**

First, sodium chloride (NaCl) (Sigma Aldrich) particles were sieved with a diameter ranging from 250-350 μm. 1.1 g of UHMWPE , 10 ml of liquid paraffin (LP) (Merk), 0.1 g of Irganox 1010 (Sigma Aldrich) and 10 g of NaCl particles were put in a glass reactor and mechanically mixed at 100rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. After that, the UHMWPE dense bar with dimensions of 15mm diameter and 50mm length (GoodFellow) was introduced 2mm depth in the NaCl/UHMWPE/LPmixture and was maintained for 10 min. Then, the glass reactor was cool down at room temperature to solidify. The NaCl/UHMWPE/LPmixture was solidified over the part of UHMWPE dense bar that was introduced in NaCl/UHMWPE/LPsolution. The solidified NaCl/UHMWPE/LP mixture and UHMWPE dense bar was removed from the glass reactor and was machined at 10mm diameter. Finally, the sample was immersed in 25mL of n-hexane and shaken under orbital agitation at room temperature for 24 h (n-hexane was changed every 12 h) to extract the LP. Then, piece was immersed in 25 mL of distilled water to extract the NaCl. These two processes were alternately run during 15 days. The porous UHMWPE sample was freeze-dried to dry completely and porous-dense UHMWPE article was obtained.

RESULTS EXAMPLE 8

From Fig. 17 it can be observed that porous UHMWPE-type polymer structure was created on the dense UHMWPE surface. It can be appreciated a continuity of the material around the dense sample contour.

**EXAMPLE 9 - Cylindrical dense-porous UHMWPE-type polymer article**

First, sodium chloride (NaCl) (Sigma Aldrich) particles were sieved with a diameter ranging from 250-350 μm. 1.1 g of UHMWPE , 10 ml of liquid paraffin (LP) (Merk), 0.1 g of Irganox 1010 (Sigma Aldrich) and 10 g of NaCl particles were put in a glass reactor and mechanically mixed at 100rpm. After the glass reactor was full of nitrogen, it was sealed. Then, the glass reactor was put in an electric heating and the mixture was heated to 160°C (30-50°C above the cloud-point temperature of the solution) for 30 min until the UHMWPE was dissolved in the LP completely. After that, the UHMWPE dense bar with dimensions of 15mm diameter and 3mm length (GoodFellow) was introduced 2mm depth in the NaCl/UHMWPE/LPmixture and was maintained for 10 min. Then, the glass reactor was cool down at room temperature to solidify. The NaCl/UHMWPE/LPmixture was solidified over the part of UHMWPE dense bar that was introduced in NaCl/UHMWPE/LPsolution. The solidified NaCl/UHMWPE/LP mixture and UHMWPE dense bar was removed from the glass reactor and was machined at 10mm diameter. Finally, the sample was immersed in 25mL of n-hexane and shaken under orbital agitation at room temperature for 24 h (n-hexane was changed every 12 h) to extract the LP. Then, piece was immersed in 25 mL of distilled water to extract the NaCl. These two processes were alternately run during 15 days. The porous UHMWPE sample was freeze-dried to dry completely and porous-dense UHMWPE article was obtained.

RESULTS EXAMPLE 9

From the SEM image (Fig. 18) it can be observed that a porous UHMWPE-type polymer structure was created on the cylindrical dense UHMWPE surface.

## Claims

1. An UHMWPE-type polymer article **characterized in that** it comprises at least a porous UHMWPE-type polymer structure, said structure comprising an at least trimodal pore distribution as follows:

   (i) a pore distribution A corresponding to pores A with an average size between 50 μm and 500 μm which are interconnected throughout the whole article;
   (ii) a pore distribution B corresponding to the voids between adjacent pores A of an average size between 5 μm and 70 μm referred to as pores B; and
   (iii) a pore distribution C corresponding to pores C of an average size of less than about 10 μm which are located in the walls of the pores A and pores B.

2. The UHMWPE-type polymer article according to claim 1, **characterized in that** it further comprises a pore distribution A' corresponding to pores A' of an average size comprised between 50-500 μm and different from pores A, which are interconnected throughout the whole article.

3. The UHMWPE-type polymer article according to claims 1 to 2, **characterized in that** the pores occupy 70 to 95% of the total volume of the porous UHMWPE-type polymer structure.

4. The UHMWPE-type polymer article according to claims 1 to 3, **characterized in that** it further comprises at least one bioactive particle in the porous UHMWPE-type polymer structure, selected from the group of metallic particles, ceramic particles or mixtures thereof..

5. The UHMWPE-type polymer article according to claims 1 to 4, **characterized in that** it further comprises a dense UHMWPE-type polymer structure to form a dense-porous UHMWPE-type polymer article.

6. A process for the production of an UHMWPE-type polymer article according to claims 1 to 5, **characterized in that** it comprises the following steps:

   a) contacting the UHMWPE-type polymer with at least one solvent and at least one porogen, wherein the percentage of UHMWPE-type polymer is between 5% to 50% by weight in respect of the solvent,
   b) mixing a UHMWPE-type polymer with at least one solvent and at least one porogen until a homogeneous mixture is obtained,
   c) heating the mixture obtained in b) to a temperature of about 30°C to about 50°C above the cloud-point temperature of the UHMWPE-type polymer /solvent/porogen mixture until complete dissolution of the UHMWPE-type polymer in the solvent,
   d) cooling the mixture obtained in c) to a temperature at which phase separation occurs,
   e) forming said cooled mixture into a shaped intermediate article,
   f) removing the solvent and the porogen agent, and
   g) recovering the article comprising an UHMWPE-type polymer.

7. The process according to claim 6 **characterized in that** in step a) the percentage of UHMWPE-type polymer is between 10% to 30% by weight in respect to the solvent.

8. The process according to claims 6 to 7, **character-**

**ized in that** the solvent is selected from diphenyl ether, diisodecyl phthalate, liquid parafine, polytetramethylene glycol, dioctyl phthalate, soybean oil, decalin, mineral oil or mixtures thereof.

**9.** The process according to claims 6 to 8, **characterized in that** the porogen is a salt-type porogen, a sugar-type porogen or mixtures thereof.

**10.** The process according to claim 9, **characterized in that** the salt-type porogen is selected from sodium chloride, sodium citrate, sodium tartrate, potassium chloride or mixtures thereof.

**11.** The process according to claim 9, **characterized in that** the sugar-type porogens is selected from sucrose, galactose or mixtures thereof.

**12.** The process according to claims 6 to 11, **characterized in that** the UHMWPE-type polymer of step a) may be further contacted with at least one bioactive particle, selected from the group of metallic particles, ceramic particles or mixtures thereof.

**13.** The process according to claims 6 to 12, **characterized in that** in step c) the mixture obtained in step b) is heated to a temperature between 150°C and 200°C.

**14.** The process according to claims 6 to 13, **characterized in that** between step c) and step d) it further comprises a step of introducing at least one surface of a dense UHMWPE-type polymer structure in the UHMWPE-type polymer/solvent/porogen mixture.

**15.** An implant and/or scaffold **characterized in that** it comprises an UHMWPE-type polymer article according to claims 1 to 5.

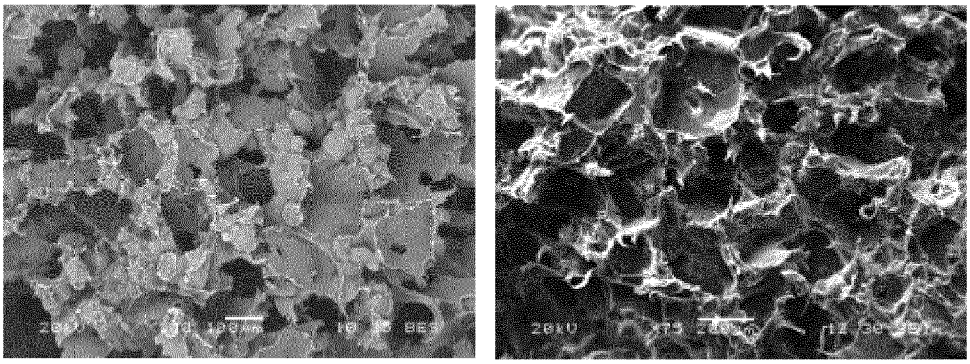

FIG.1

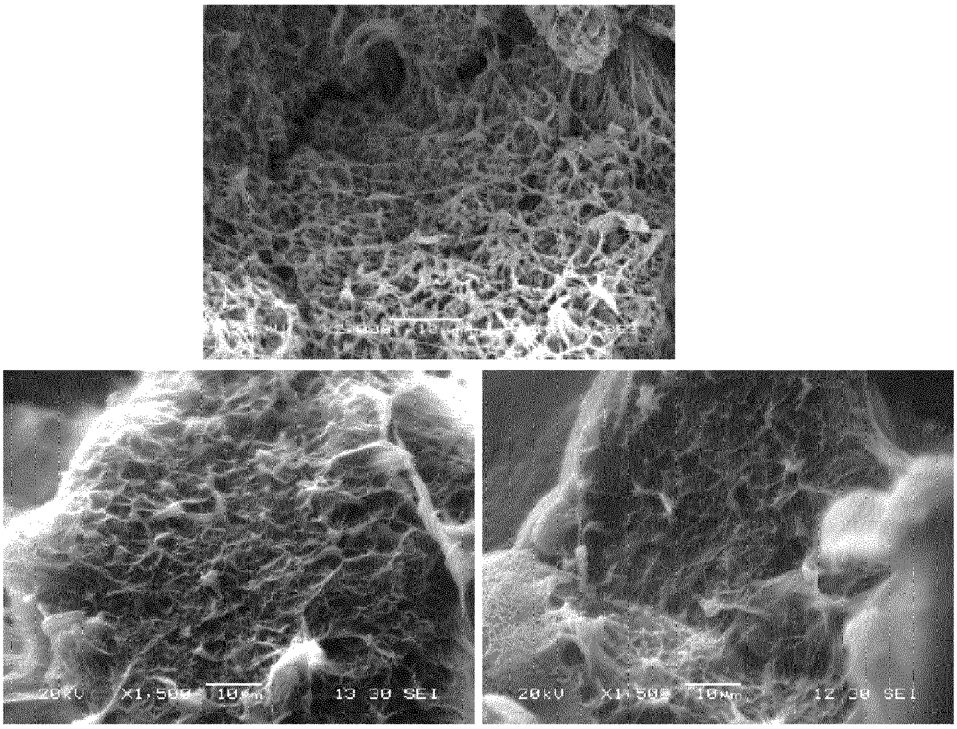

FIG. 2

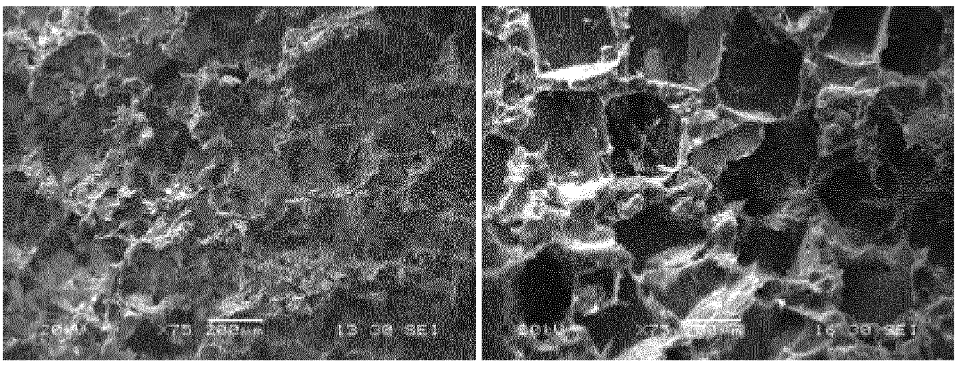

FIG.3

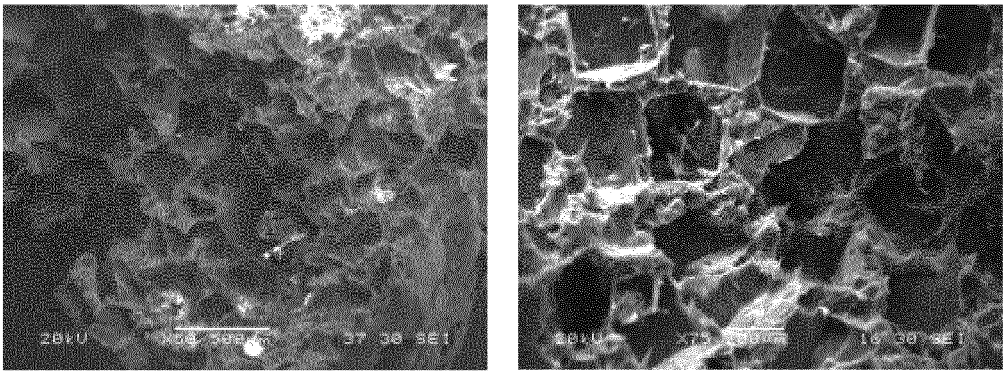

FIG. 4

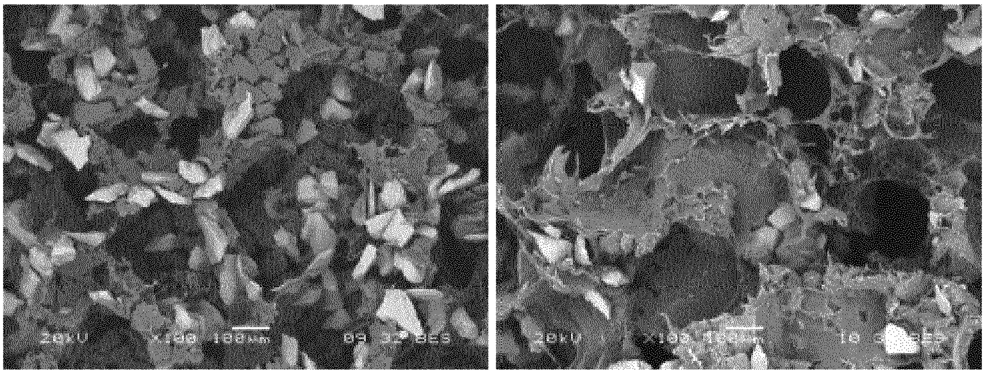


FIG. 5

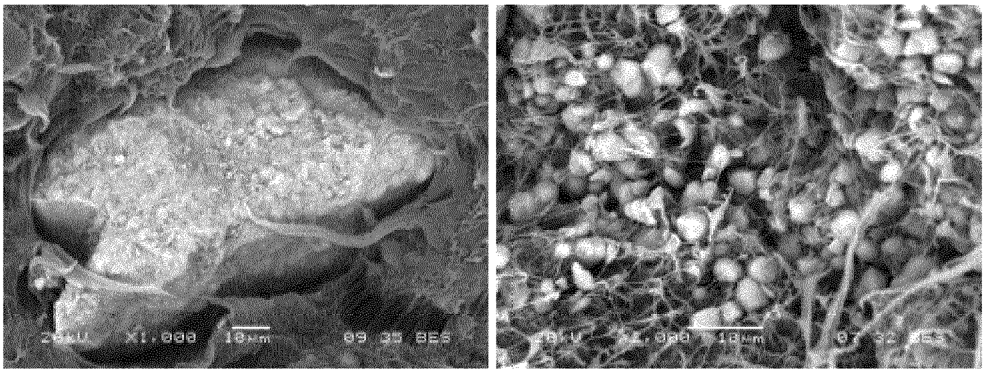


FIG. 6

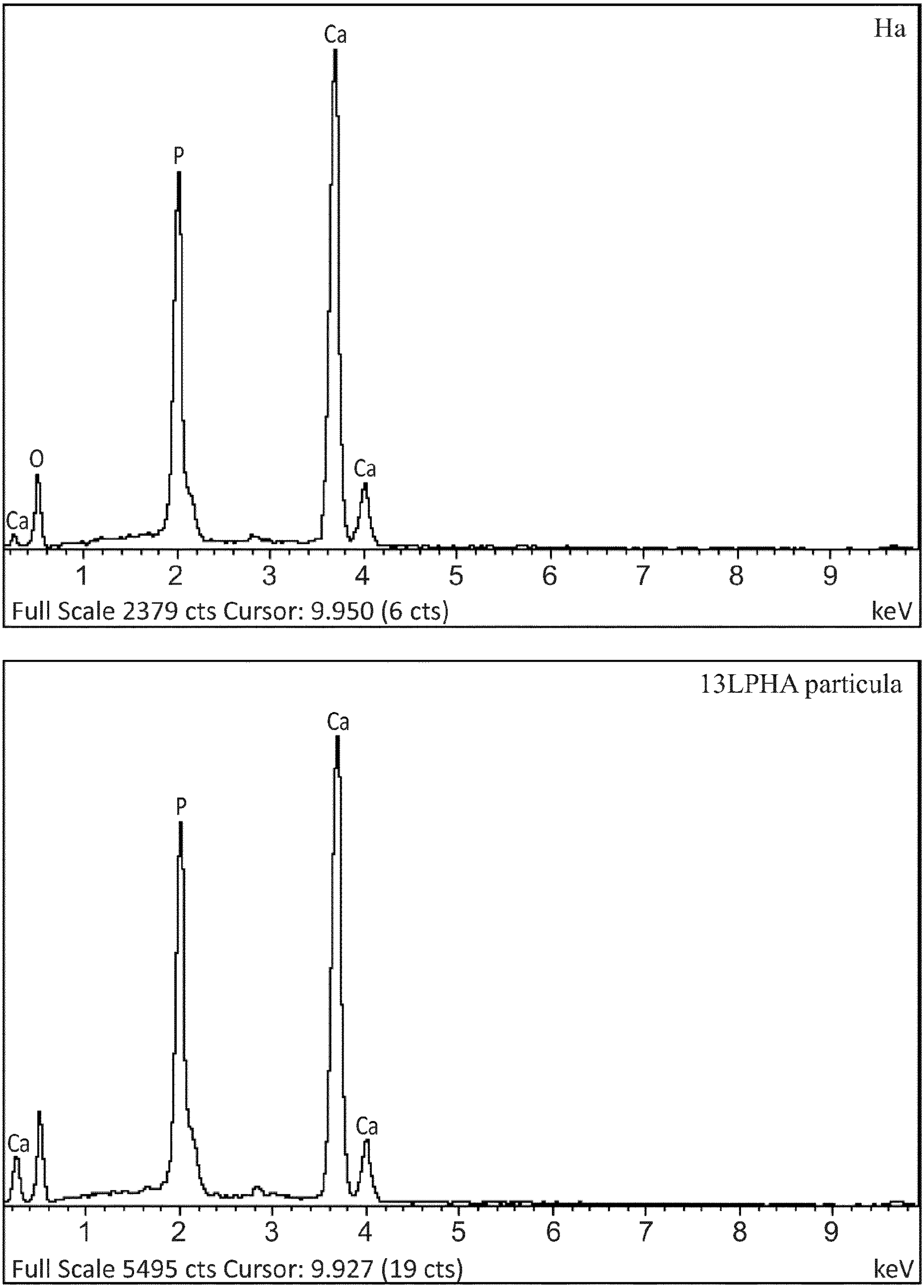
Ha
Ca
P
O
Ca
Ca
1 2 3 4 5 6 7 8 9
Full Scale 2379 cts Cursor: 9.950 (6 cts)
keV
13LPHA particula
Ca
P
Ca
Ca
1 2 3 4 5 6 7 8 9
Full Scale 5495 cts Cursor: 9.927 (19 cts)
keV

**FIG. 7**

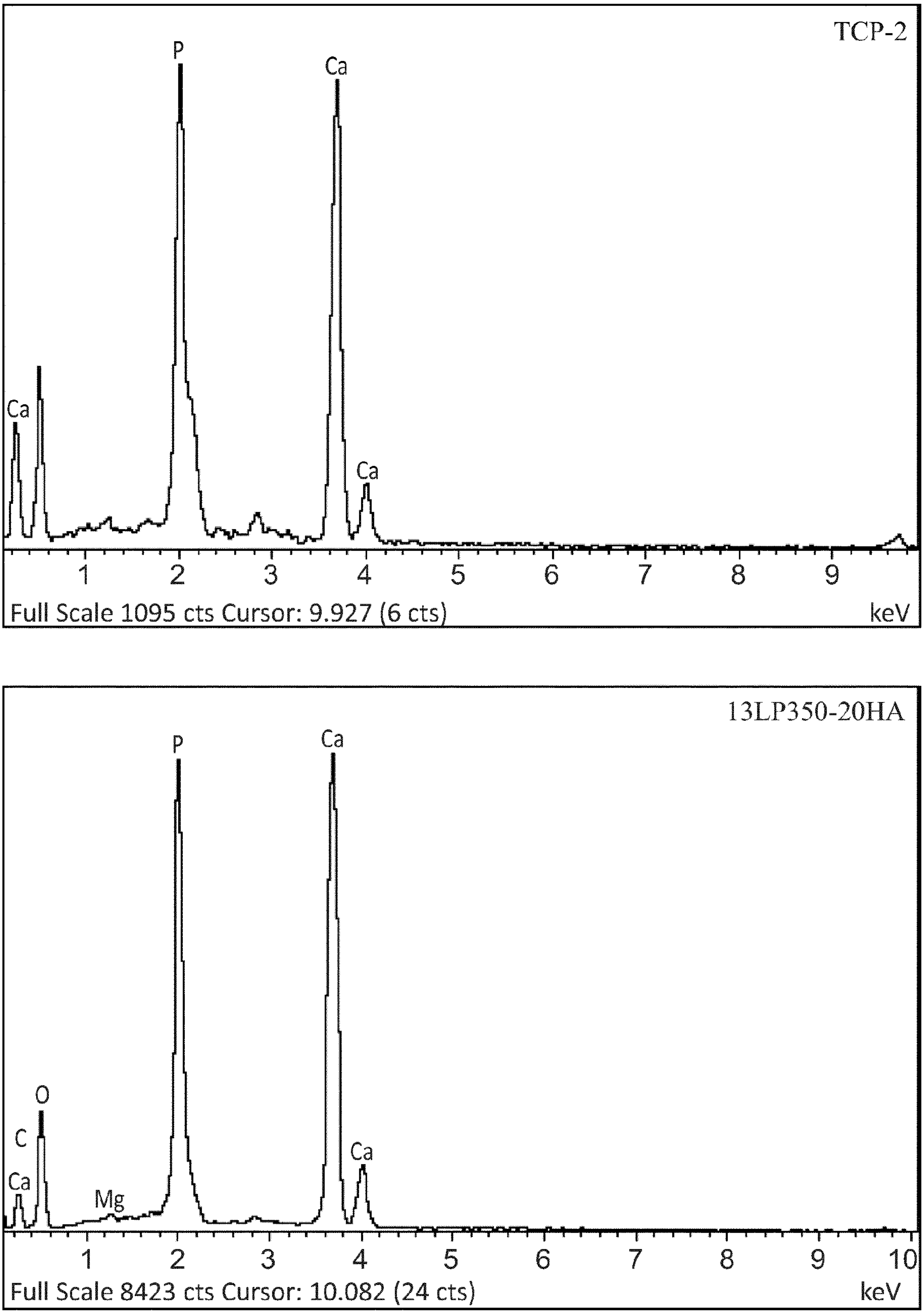
TCP-2
P
Ca
Ca
Ca
1 2 3 4 5 6 7 8 9
Full Scale 1095 cts Cursor: 9.927 (6 cts)
keV
13LP350-20HA
P
Ca
O
C
Ca
Mg
Ca
1 2 3 4 5 6 7 8 9 10
Full Scale 8423 cts Cursor: 10.082 (24 cts)
keV

**FIG. 8**

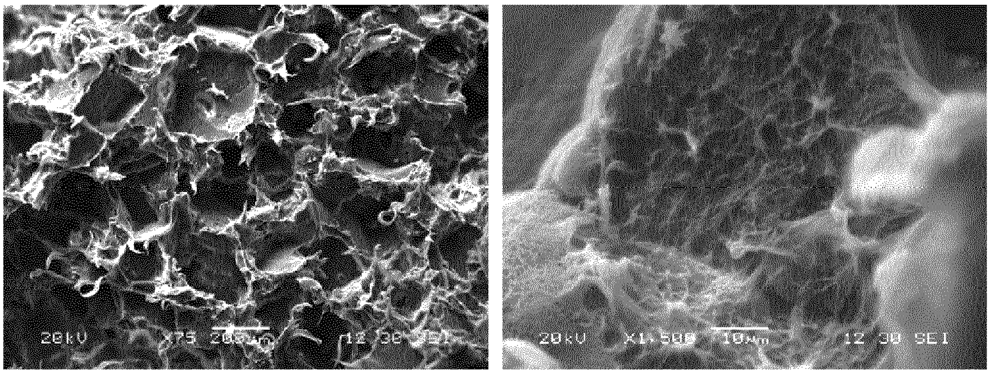
20kV X75
20kV X1,500 10µm 12 30 SEI

FIG.9

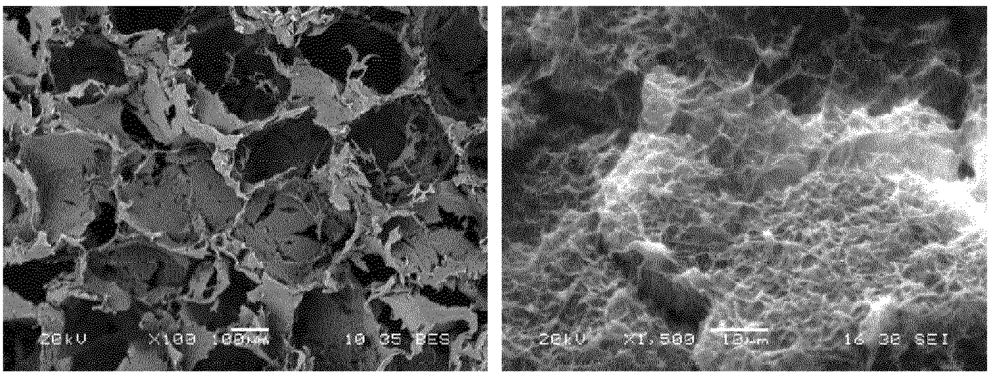
20kV X100 100µm 10 35 BES
20kV X1,500 10µm 16 30 SEI

FIG. 10

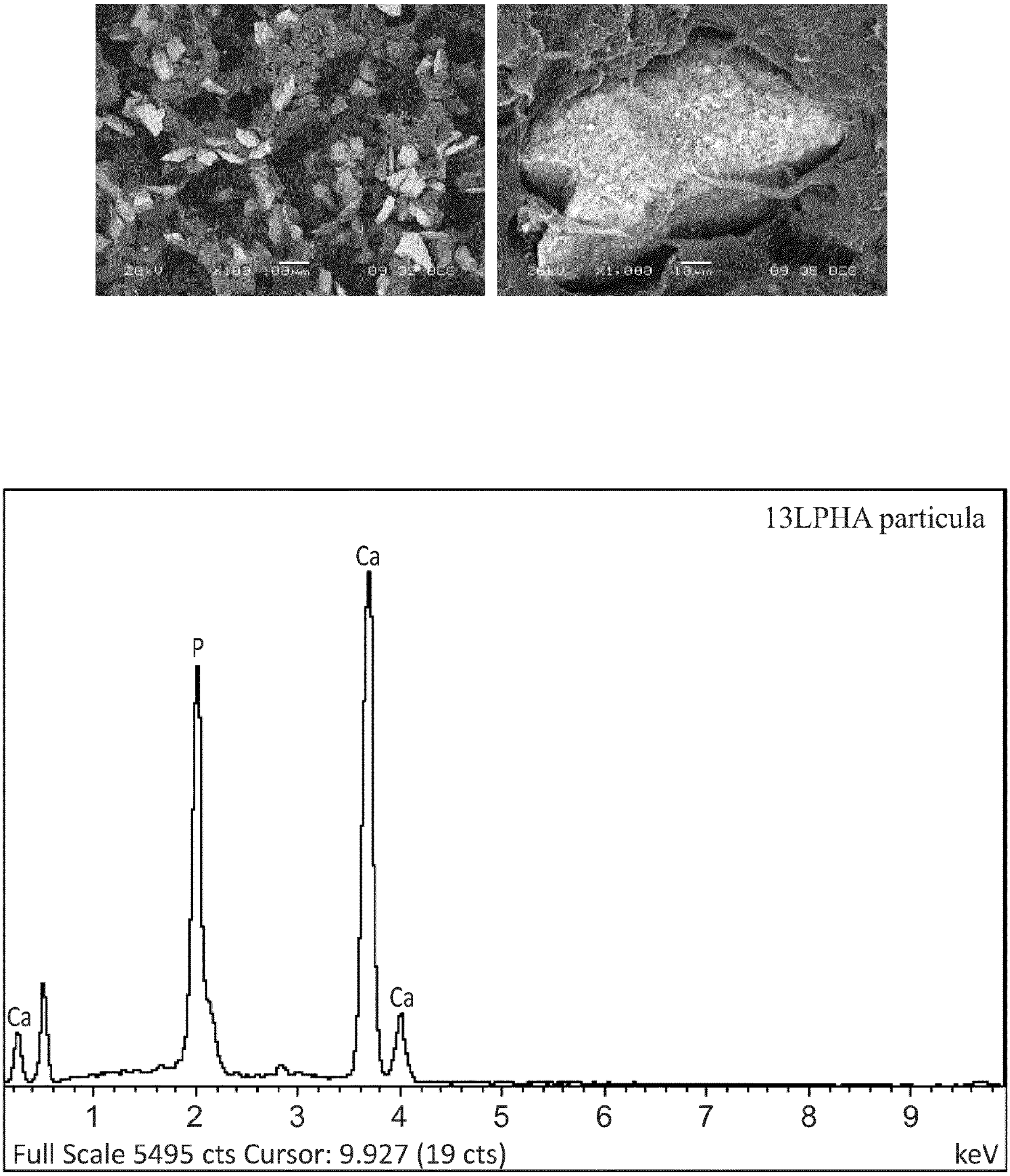
13LPHA particula
Ca
P
Ca
Ca
1
2
3
4
5
6
7
8
9
Full Scale 5495 cts Cursor: 9.927 (19 cts)
keV

**FIG. 11**

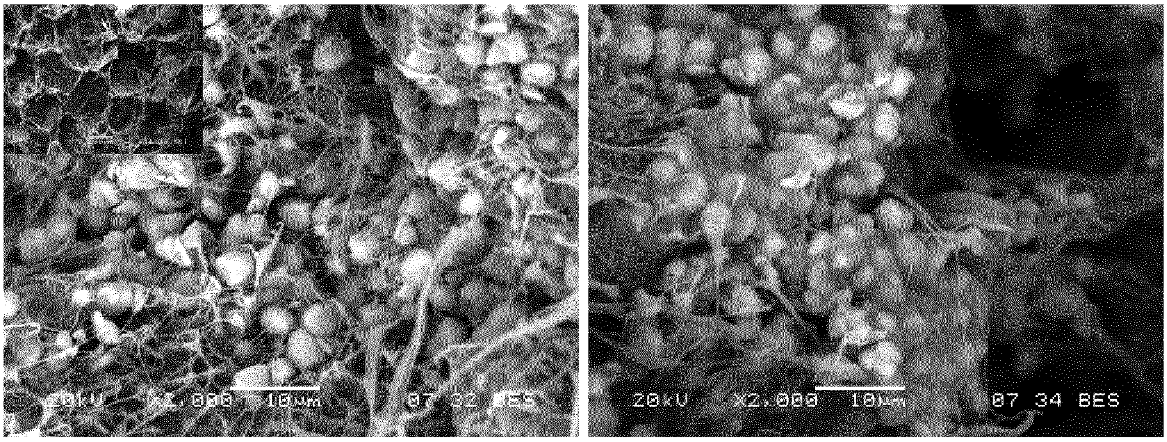


FIG. 12

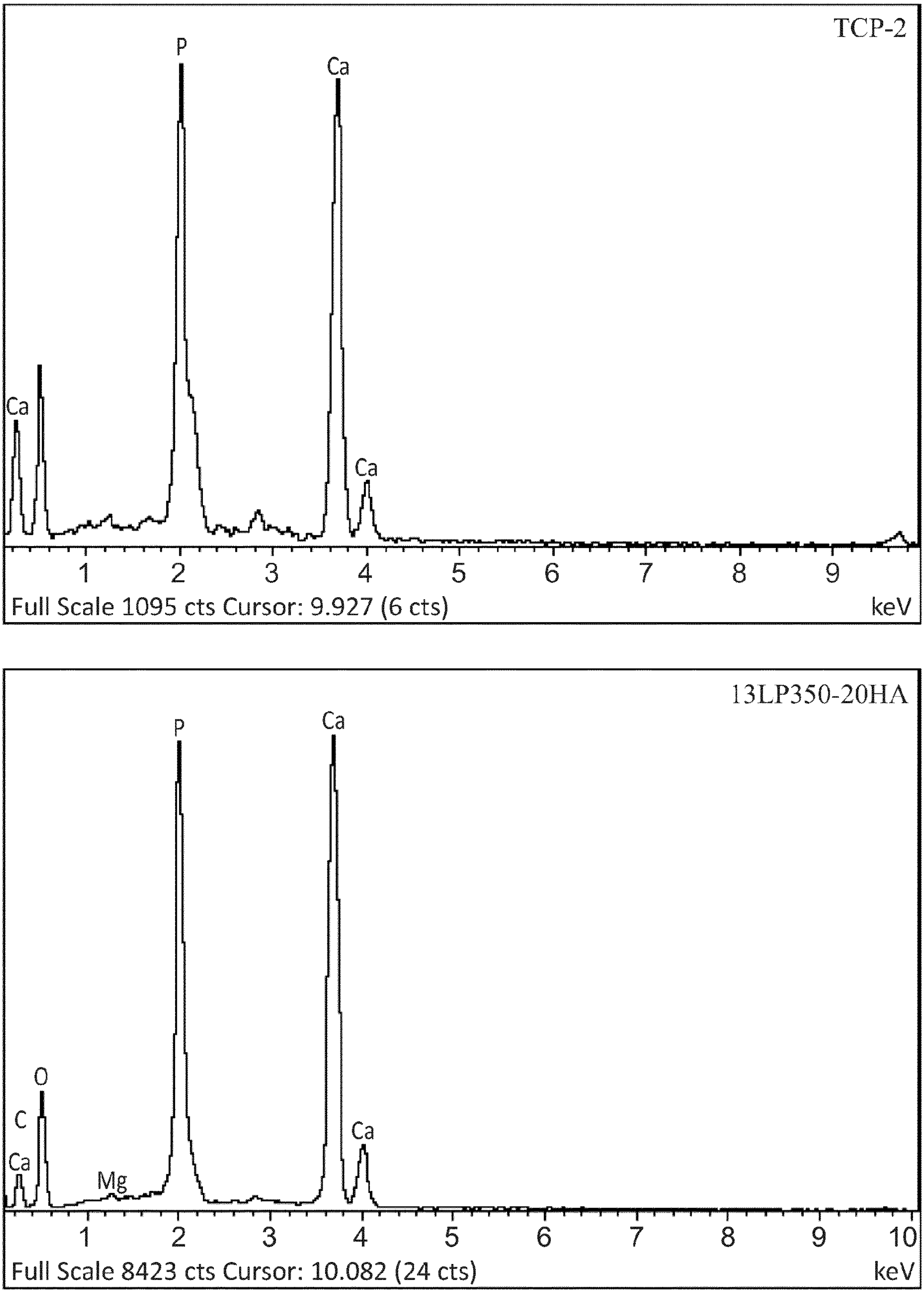
TCP-2
P
Ca
Ca
Ca
1 2 3 4 5 6 7 8 9
Full Scale 1095 cts Cursor: 9.927 (6 cts)
keV
13LP350-20HA
P
Ca
O
C
Ca
Mg
Ca
1 2 3 4 5 6 7 8 9 10
Full Scale 8423 cts Cursor: 10.082 (24 cts)
keV

FIG. 13

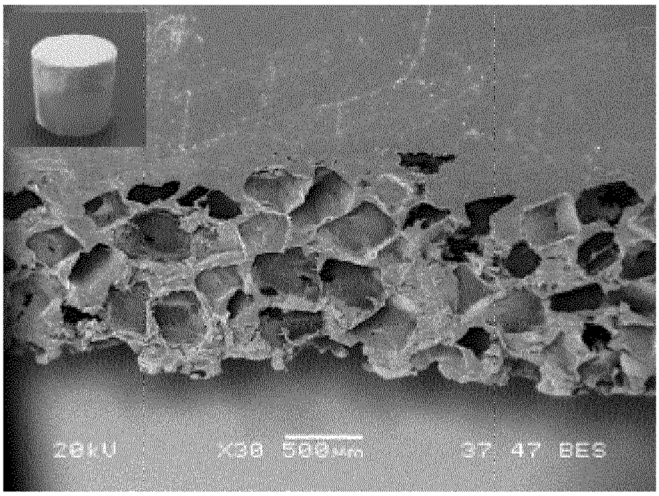


FIG. 14

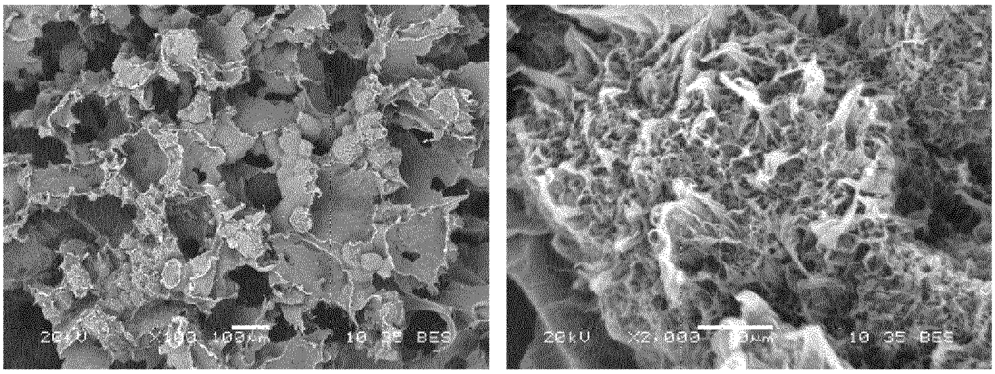


FIG. 15

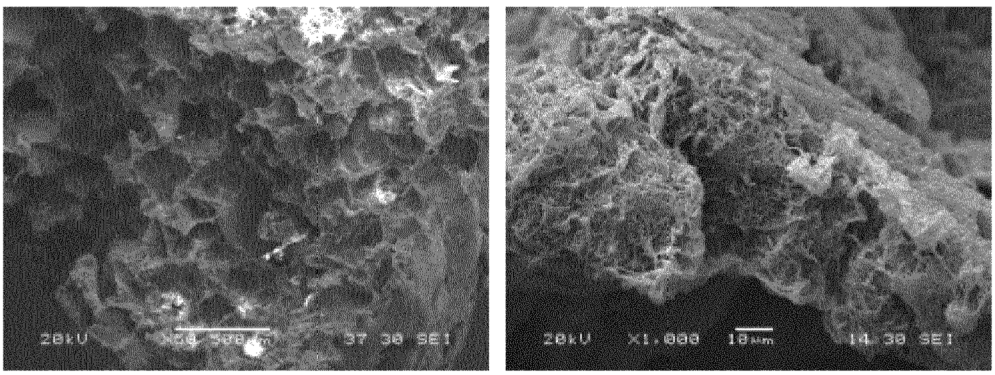


FIG. 16

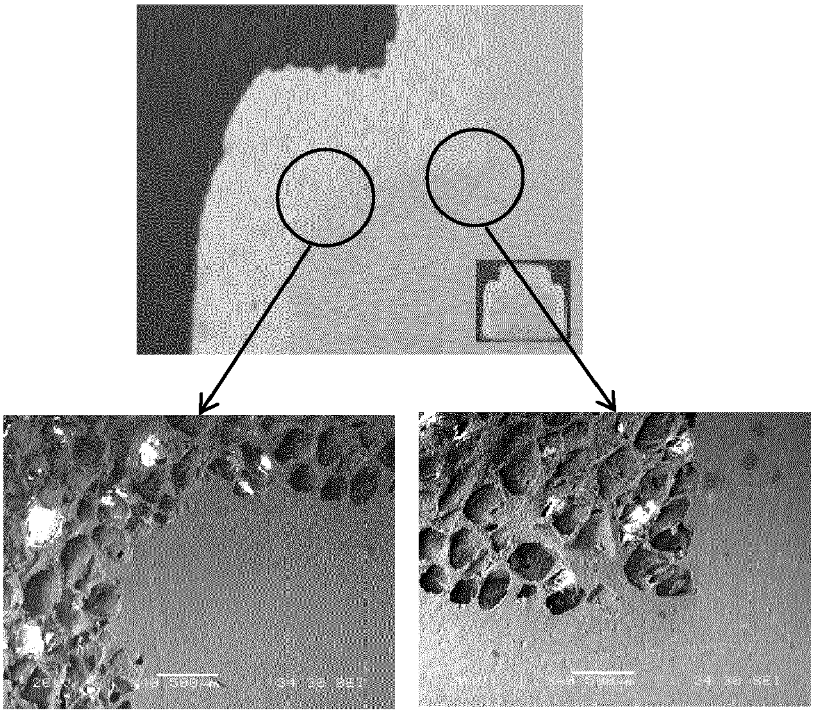

FIG. 17

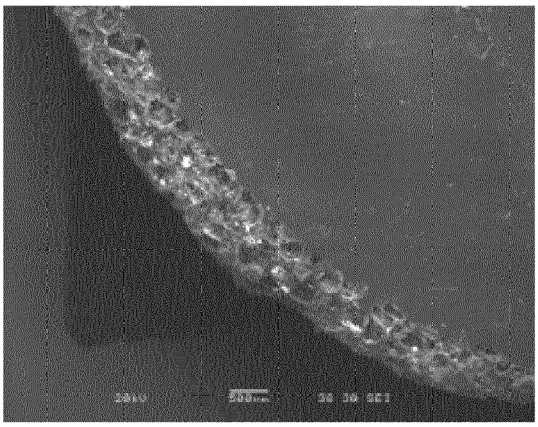

FIG. 18

Europäisches Patentamt
European Patent Office
Office européen des brevets

# EUROPEAN SEARCH REPORT

Application Number
EP 15 38 2675

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 468 703 A1 (DEPUY PRODUCTS INC [US]) 20 October 2004 (2004-10-20) * claims; examples * ----- | 1-15 | INV. A61L27/16 A61L27/56 |
| A | EP 2 238 992 A1 (NGK SPARK PLUG CO [JP]) 13 October 2010 (2010-10-13) * paragraphs [0028] - [0042]; claims; figure 1; examples * ----- | 1-15 | |
| X | EP 2 338 532 A2 (FUNDACION INASMET [ES]) 29 June 2011 (2011-06-29) * claims * ----- | 1-15 | |

TECHNICAL FIELDS SEARCHED (IPC)

A61L

The present search report has been drawn up for all claims

2

| Place of search | Date of completion of the search | Examiner |
|---|---|---|
| The Hague | 8 June 2016 | Messemanne, Jasmine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 38 2675

This annex lists the patent family members relating to the patent documents cited in the above-mentioned European search report. The members are as contained in the European Patent Office EDP file on
The European Patent Office is in no way liable for these particulars which are merely given for the purpose of information.

08-06-2016

| Patent document cited in search report | Publication date | Patent family member(s) | Publication date |
|---|---|---|---|
| EP 1468703 A1 | 20-10-2004 | AT 477008 T | 15-08-2010 |
| | | AU 2004201549 A1 | 04-11-2004 |
| | | DK 1468703 T3 | 08-11-2010 |
| | | EP 1468703 A1 | 20-10-2004 |
| | | EP 2191852 A2 | 02-06-2010 |
| | | ES 2347963 T3 | 26-11-2010 |
| | | JP 4767500 B2 | 07-09-2011 |
| | | JP 2004313794 A | 11-11-2004 |
| | | US 2004210316 A1 | 21-10-2004 |
| EP 2238992 A1 | 13-10-2010 | EP 2238992 A1 | 13-10-2010 |
| | | EP 2737912 A1 | 04-06-2014 |
| | | JP 5372782 B2 | 18-12-2013 |
| | | US 2011022181 A1 | 27-01-2011 |
| | | WO 2009095960 A1 | 06-08-2009 |
| EP 2338532 A2 | 29-06-2011 | CA 2785571 A1 | 30-06-2011 |
| | | EP 2338532 A2 | 29-06-2011 |
| | | US 2012323339 A1 | 20-12-2012 |
| | | WO 2011076971 A2 | 30-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010283184 A1
- US 2014052264 A1
- WO 2014120977 A1
- US 2004019389 A1


### Non-patent literature cited in the description


- **DING H. et al.** *Journal of Applied Polymer Science,* 2007, vol. 105, 3355-3362